# EUROPÄISCHE PATENTSCHRIFT

(11) **EP 0 467 909 B1**
(45) Veröffentlichungstag und Bekanntmachung des Hinweises auf die Patenterteilung: **23.06.1993**
(21) Anmeldenummer: 90905479.3
(22) Anmeldetag: 07.04.1990
(51) Int. Cl.: A61C 1/00

(54) **VORRICHTUNG ZUM ZERSTÖREN UND ABTRAGEN VON ZAHNMATERIAL**
APPARATUS FOR DESTROYING AND REMOVING MATERIAL FROM TEETH
APPAREIL POUR DETRUIRE ET ELIMINER DE LA MATIERE DENTAIRE

(30) Priorität: 11.04.1989 DE 3911871
(43) Veröffentlichungstag der Anmeldung: 29.01.1992
(73) Patentinhaber: AESCULAP AG, D-78501 Tuttlingen (DE)
(72) Erfinder: STEINER, Rudolf, D-7900 Ulm (DE); KELLER, Ulrich, D-7904 Dellmensingen (DE); HIBST, Raimund, D-7904 Erbach (DE)
(74) Vertreter: Hoeger, Stellrecht & Partner
(86) Internationale Anmeldenummer: EP9000554
(87) Internationale Veröffentlichungsnummer: WO9011728

(56) Entgegenhaltungen:
- EP-A- 0 073 617
- DE-A- 3 415 293

## Beschreibung

Die Erfindung betrifft eine Vorrichtung zum Zerstören und Abtragen von Zahnmaterial mittels gepulster Laserstrahlen mit einem Laser zur Erzeugung gepulster Laserstrahlen, einem Laserhandstück, einer Flüssigkeitszufuhr und einer Flüssigkeit.

Es ist bekannt, daß mit gepulsten Er:YAG-Laserstrahlen Zahngewebe ähnlich wie mit mechanischen Bohrern abgetragen werden kann, wobei sich dabei sowohl im Schmelz als auch im Dentin Krater mit einer minimalen Schädigung der Umgebung ergeben. Die Abtragung von Dentin ist dabei effektiver als die Abtragung von Schmelz, besonders effektiv läßt sich kariöses Gewebe entfernen (R. Hibst et al in "Laser in Medicine and Surgery" - MZV-Verlag 4: 163-165 (1988)).

Es hat sich aber bei licht- und elektronenmikroskopischen Untersuchungen herausgestellt, daß zwar bei Verwendung der genannten gepulsten Infrarot-Laserstrahlung Risse und Verglasungen des an den Krater angrenzenden Materials vermieden werden, daß aber trotzdem im Dentin eine leichte bräunliche Verfärbung des Kraterrandes auftritt.

Andererseits sind Laserhandstücke bekannt, bei denen der von der auftretenden Laserstrahlung beaufschlagte Bereich des Zahnmateriales alternierend mit den Laserpulsen mit Wasser besprüht wird, wobei die alternierende Besprühung wesentlich ist, da festgestellt worden ist, daß der Laser in einer Wasserdampfatmospähre nicht wirksam funktioniert (EP-A-0073617).

Es ist Aufgabe der Erfindung, eine gattungsgemäße Vorrichtung derart zu verbessern, daß eine noch größere Schonung des verbleibenden Gewebes gewährleistet ist, ohne daß die Effektivität der Materialabtragung bei der Laserbestrahlung herabgesetzt wird.

Diese Aufgabe wird bei einer Vorrichtung der eingangs beschriebenen Art erfindungsgemäß dadurch gelöst, daß der Laser ein Infrarot-Laser ist, daß diese Flüssigkeit im Bereich der Wellenlänge der Infrarot-Laserstrahlung absorbiert und daß vor oder während der Bestrahlung die Flüssigkeit auf das zu bearbeitende Zahnmaterial in einer Schichtdicke zwischen 10 und 200 µm aufsprühbar ist.

Überraschenderweise hat sich herausgestellt, daß durch diese dünne, an sich die Laserstrahlung absorbierende Schicht nicht nur keine Schwächung der Wirkung der Laserstrahlung bei der Erzeugung der Krater erfolgt, sondern gleichzeitig das umliegende Gewebe praktisch vollständig unversehrt bleibt. Eine bräunliche Verfärbung des Dentins im Bereich des Kraterrandes tritt nicht mehr auf, und trotzdem läßt sich das Material im unmittelbaren Bestrahlungsbereich mit derselben Effektivität abtragen wie ohne die Verwendung der Flüssigkeit. Der Wirkungsmechanismus ist noch nicht in vollem Umfange geklärt, jedoch führt möglicherweise die Expansion des bei der Bestrahlung entstehenden Dampfes der absorbierenden Flüssigkeit zu einer besonders effektiven, lokalen Abkühung des die Bohrstelle umgebenden Gewebes.

Als Flüssigkeit wird vorzugsweise Wasser verwendet, die Flüssigkeit kann Kochsalz und/oder Detergentien oder andere Zusätze enthalten.

Besonders vorteilhaft ist es, wenn die pro Zeiteinheit auf das Zahnmaterial aufgebrachte Flüssigkeitsmenge erhöht wird, wenn die Strahlungsenergie por Laserpuls erhöht wird. Mit anderen Worten werden auf das Zahnmaterial aufgebrachte Flüssigkeitsmenge und pro Zeiteinheit aufgebrachte Strahlungsenergie in gleicher Weise erhöht und herabgesetzt, um auf diese Weise sicherzustellen, daß die erhöhte Verdampfungsrate der Flüssigkeit beim Auftreffen einer größeren Strahlungsenergie ausgeglichen wird. Dadurch ist es möglich, die Schichtdicke vor dem Auftreffen eines Laserpulses im wesentlichen konstant zu halten, und zwar unabhängig davon, wie groß die beim vorhergehenden Laserpuls verdampfte Flüssigkeitsmenge ist.

Besonders vorteilhaft ist es, wenn man die Flüssigkeit intermittierend vor jedem Laserpuls aufträgt. Es ist damit gewährleistet, daß vor den Laserpulsen, die etwa mit einer Repetitionsrate von 1 Hz erzeugt werden, wieder eine nicht erwärmte, neue Flüssigkeitsschicht aufgetragen wird.

Es ist aber natürlich auch möglich, daß man die Flüssigkeit kontinuierlich aufträgt.

Bei der kontinuierlichen Auftragung ist es weiterhin günstig, wenn man die pro Zeiteinheit auf das Zahnmaterial aufgebrachte Flüssigkeitsmenge erhöht, wenn man die Repetitionsrate der Laserpulse erhöht, und umgekehrt. Eine Erhöhung der Repetitionsrate der Laserpulse erhöht die auf das Zahnmaterial pro Zeiteinheit auftreffende Strahlungsenergie und damit auch die pro Zeiteinheit verdampfte Flüssigkeit. Um eine konstante Flüssigkeitsbedeckung des Zahnmaterials zu gewährleisten, ist es daher vorteilhaft, in entsprechender Weise eine erhöhte Flüssigkeitsmenge auf das Zahnmaterial aufzubringen.

Eine besonders gleichmäßige Verteilung des Flüssigkeitsfilmes mit gleichmäßiger Schichtdicke erhält man, wenn man die Flüssigkeit in Form eines Sprühnebels auf das Zahnmaterial aufbringt.

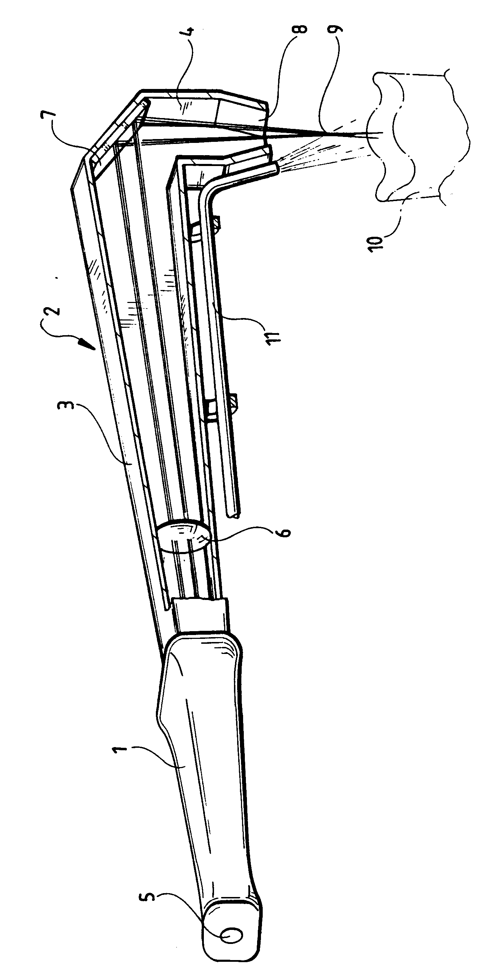

Die nachfolgende Beschreibung einer bevorzugten Ausführungsform eines zur Laserbehandlung geeigneten Handstückes dient im Zusammenhang mit der Zeichnung der näheren Erläuterung.

Das in der Zeichnung dargestellte Handstück umfaßt einen Griff 1 und ein L-förmiges Gehäuse 2 mit zwei senkrecht zueinander stehenden Schenkeln 3 beziehungsweise 4. Der eine Schenkel 3 schließt sich unmittelbar an den Griff 1 an, der andere Schenkel 4 steht an dem dem Griff 1 abgewandten Ende des Schenkels 3 senkrecht von diesem nach unten ab.

Im Inneren des Griffs 1 befindet sich ein in der Zeichnung nicht dargestellter Lichtleiter, beispielsweise ein Glasfaserleiter, der über einen Anschluß 5 an einen in der Zeichnung ebenfalls nicht dargestellten Lichtleiter anschließbar ist. Über diese Lichtleiter wird dem Handgriff die gepulste Infrarotstrahlung eines Er:YAG-Lasers mit einer Wellenlänge von 2,94 Mikrometern zugeleitet, beispielsweise mit einer Impulsdauer von 200 Mikrosekunden, Strahlungsenergien von 10 bis 1000 mJ pro Puls und eine Repetitionsrate von 1/2 bis 5 Hz. Diese Behandlungsstrahlung wird in einer am griffseitigen Ende des Gehäuses 2 angeordneten Konvexlinse 6 gebündelt und über einen im Übergangsbereich zwischen den beiden Schenkeln 3 und 4 angeordneten, unter 45° schräggestellten Umlenkspiegel 7 durch eine am freien Ende des Schenkels 4 angeordnete Austrittsöffnung 8 aus dem Gehäuse 2 herausgeführt. In geringem Abstand von der Austrittsöffnung, beispielsweise im Abstand zwischen 5 und 20 mm, wird die Behandlungsstrahlung fokussiert, so daß der Behandlungsstrahl 9 im fokussierten Bereich auf das zu behandelnde Material eines Zahnes 10 auffallen kann.

An der Unterseite des Gehäuses 2 befindet sich eine Zufuhrleitung 11 für eine Flüssigkeit, die auf den für die Behandlung vorgesehenen Bereich des Zahnes 10 gerichtet ist und Flüssigkeit in Form eines Sprühnebels so auf den Zahn sprüht, daß der Auftreffbereich des Behandlungsstrahles mit einem dünnen und zusammenhängenden Flüssigkeitsfilm überzogen wird.

Diese Flüssigkeit kann Wasser oder eine andere Flüssigkeit sein, die im Wellenlängenbereich der Behandlungsstrahlung absorbiert, die also den Behandlungsstrahl absorbiert. Die Wirkung kann auch durch Zusätze zu der Flüssigkeit verbessert werden, beispielsweise durch Zusätze von Detergentien wie Natriumlaurylsulfat, die für eine gleichmäßige Ausbreitung des Flüssigkeitsfilms sorgen..

Das Aufbringen der Flüssigkeit erfolgt in Form eines Sprühnebels entweder kontinuierlich oder vorzugsweise intermittierend im Takt der Pulsation des Behandlungsstrahles, so daß jeweils vor einem Behandlungsstrahlungspuls ein Flüssigkeitspuls auf die zu behandelnde Fläche des Zahns gelangt.

Die Schichtdicke des vor jedem Strahlungspuls aufgebrachten Wasserfilms sollte zwischen 10 und 200 Mikrometern liegen, vorzugsweise im Bereich zwischen 30 und 60 Mikrometern.

Es hat sich herausgestellt, daß dieser Flüssigkeitsfilm zwar die Behandlungsstrahlung absorbiert, daß jedoch der absorbierte Anteil Bo gering ist, daß die Materialabtragung im gewünschten Bereich praktisch nicht herabgesetzt wird. Andererseits ergibt sich aber im angrenzenden Bereich eine geringere Erwärmung und geringere Beanspruchung des Materials, so daß dieses bereits unmittelbar am entstehenden Kraterrand im wesentlichen unbeschädigt zurückbleibt. Gegenüber einem nicht mit einer absorbierenden Flüssigkeit beschichteten Zahnmaterial ergibt sich somit eine wesentlich höhere Qualität der Abtragung bei gleicher Abtragungsrate.

Die Repetitionsrate der Laserpulse läßt sich verändern. Wenn die Flüssigkeit pulsierend im Takt der Laserpuls-Repetitionsrate aufgebracht wird, ist sichergestellt, daß auch bei veränderter Repetitionsrate immer eine Flüssigkeitsschicht mit annähernd gleicher Dicke das Zahnmaterial bedeckt, bevor ein Laserpuls auf das Zahnmaterial auftrifft.

Wird dagegen die Flüssigkeit kontinuierlich aufgetragen, ist es günstig, bei einer Änderung der Laserpuls-Repetitionsrate auch die pro Zeiteinheit aufgetragene Flüssigkeitsmenge entsprechend zu ändern, um entsprechend der geänderten zeitlichen Energiedichte der auffallenden Strahlung und der damit unterschiedlichen Abdampfrate der Flüssigkeit pro Zeiteinheit immer eine kontinuierliche Schichtdicke aufrechtzuerhalten.

Es ist weiterhin möglich, den Energieinhalt eines Laserpulses zu verändern, beispielsweise durch Veränderung der Laserpulsdauer. Auch in diesem Fall ist es günstig, wenn entsprechend der Änderung der Energie eines Laserpulses die Auftragrate für die Flüssigkeit angepaßt wird, so daß die entsprechend geänderte Abdampfrate gerade ausgeglichen wird. Dadurch läßt sich eine Flüssigkeitsbedeckung des Zahnmaterials mit annähernd konstanter Schichtdicke erreichen.

## Patentansprüche

1. Vorrichtung zum Zerstören und Abtragen von Zahnmaterial mittels gepulster Laserstrahlen (9) mit einem Laser zur Erzeugung gepulster Laserstrahlen (9), einem Laserhandstück (1, 2), einer Flüssigkeitszufuhr (11) und einer Flüssigkeit, dadurch gekennzeichnet, daß der Laser ein Infrarot-Laser ist, daß die Flüssigkeit im Bereich der Wellenlänge der Infrarot-Laserstrahlung absorbiert und daß vor oder während der Bestrahlung die Flüssigkeit auf das zu bearbeitende Zahnmaterial (10) in einer Schichtdicke zwischen 10 und 200 µm aufsprühbar ist.

2. Vorrichtung nach Anspruch 1, dadurch gekennzeichnet, daß die Flüssigkeit Wasser ist.

3. Vorrichtung nach Anspruch 1 oder 2, dadurch gekennzeichnet, daß die Flüssigkeit Detergentien enthält.

4. Vorrichtung nach einem der voranstehenden Ansprüche, dadurch gekennzeichnet, daß die pro Zeiteinheit abgegebene Flüssigkeitsmenge erhöht wird, wenn die Strahlungsernergie pro Laserpuls erhöht wird.

5. Vorrichtung nach einem der voranstehenden Ansprüche, dadurch gekennzeichnet, daß es die Flüssigkeit intermittierend vor jedem Laserpuls abgibt.

6. Vorrichtung nach einem der Ansprüche 1 bis 4, dadurch gekennzeichnet, daß es die Flüssigkeit kontinuierlich abgibt.

7. Vorrichtung nach Anspruch 6, dadurch gekennzeichnet, daß es die pro Zeiteinheit abgegebene Flüssigkeitsmenge erhöht, wenn die Repetitionsrate der Laserpulse erhöht wird.

8. Vorrichtung nach einem der voranstehenden Ansprüche, dadurch gekennzeichnet, daß es die Flüssigkeit in Form eines Sprühnebels abgibt.

## Claims

1. Apparatus for destroying and removing material from teeth by means of pulsed laser beams (9), comprising a laser for generating pulsed laser beams (9), a laser handpiece (1, 2), a liquid supply means (11) and a liquid, characterized in that the laser is an infrared laser, that the liquid absorbs in the range of the wavelength of the infrared laser radiation and that prior to or during the irradiation the liquid is sprayable onto the tooth material (10) to be treated in a layer having a thickness of between 10 and 200 µm.

2. Apparatus as defined in claim 1, characterized in that the liquid is water.

3. Apparatus as defined in claim 1 or 2, characterized in that the liquid contains detergents.

4. Apparatus as defined in any of the preceding claims, characterized in that the amount of liquid delivered per time unit is increased when the radiation energy per laser pulse is increased.

5. Apparatus as defined in any of the preceding claims, characterized in that it delivers the liquid intermittently prior to each laser pulse.

6. Apparatus as defined in any of claims 1 to 4, characterized in that it delivers the liquid continuously.

7. Apparatus as defined in claim 6, characterized in that it increases the amount of liquid delivered per time unit when the rate of repetition of the laser pulses is increased.

8. Apparatus as defined in any of the preceding claims, characterized in that it delivers the liquid in the form of a spray.

## Revendications

1. Dispositif de destruction et d'enlèvement de matière dentaire au moyen de faisceaux lasers pulsés (9) comportant un laser pour l'émission de faisceaux lasers pulsés (9), une pièce à main (1, 2), une amenée de liquide (11) et un liquide, caractérisé en ce que le laser est un laser infrarouge, en ce que le liquide est absorbant dans le domaine de longueur d'ondes du rayonnement laser infrarouge et en ce que le liquide est pulvérisable sur la matière dentaire (10) à traiter en une couche d'épaisseur comprise entre 10 et 200 µm avant ou pendant l'irradiation.

2. Dispositif selon la revendication 1, caractérisé en ce que le liquide est de l'eau.

3. Dispositif selon la revendication 1 ou 2, caractérisé en ce que le liquide contient des détergents.

4. Dispositif selon une des revendications précédentes, caractérisé en ce que la quantité de liquide fournie par énergie de temps est accrue lorsqu'est accrue l'énergie rayonnante par impulsion laser.

5. Dispositif selon une des revendications précédentes, caractérisé en ce que le liquide est fourni de façon intermittente avant chaque impulsion laser.

6. Dispositif selon une des revendications 1 à 4, caractérisé en ce que le liquide est fourni de façon continue.

7. Dispositif selon la revendication 6, caractérisé en ce que la quantité de liquide fournie par unité de temps est accrue lorsque la fréquence de répétition des impulsions lasers est accrue.

8. Dispositif selon une des revendications précédentes, caractérisé en ce que le liquide est fourni sous la forme d'un brouillard de fines gouttelettes.
